# EUROPÄISCHE PATENTSCHRIFT

(11) **EP 1 207 913 B1**
(45) Veröffentlichungstag und Bekanntmachung des Hinweises auf die Patenterteilung: **13.07.2005**
(21) Anmeldenummer: 00974272.7
(22) Anmeldetag: 28.08.2000
(51) Int. Cl.: A61L 9/04, A61L 9/12

(54) **ANORDNUNG ZUR LUFTVERBESSERUNG**
DEVICE FOR DEODORIZING AIR
SYSTEME POUR DESODORISER L'AIR

(30) Priorität: 30.08.1999 DE 19941200; 30.08.1999 DE 29915186 U
(43) Veröffentlichungstag der Anmeldung: 29.05.2002
(73) Patentinhaber: Mutschlechner, Ilona, 51580 Reichshof (DE)
(72) Erfinder: Mutschlechner, Ilona, 51580 Reichshof (DE)
(74) Vertreter: Brandenburg, Thomas, Dr.
(86) Internationale Anmeldenummer: PCT/DE2000/002929
(87) Internationale Veröffentlichungsnummer: WO 2001/015748

(56) Entgegenhaltungen:
- EP-A- 0 835 666
- FR-A- 1 601 586
- GB-A- 2 194 440
- US-A- 5 170 886

## Beschreibung

Die Erfindung betrifft eine Anordnung zur Luftverbesserung. Derartige Anordnungen sind beispielsweise als Duftspray oder Duftkissen zur Duftstoffabgabe bekannt und finden im gewerblichen wie auch privaten Bereich Anwendung. Ferner betrifft die Erfindung ein Verfahren zur Herstellung einer Luftverbesserungsanordnung.

Es ist eine Aufgabe der Erfindung, eine Luftverbesserungsanordnung bereitzustellen, die vielseitig einsetzbar ist und sich in einfacher Weise herstellen läßt.

Die US-A-4,600,146 offenbart einen Körper zur beständigen und langsamen Abgabe eines in dem Körper eingeschlossenen Mittels, wobei der Körper eine zylindrische Röhre aus einem flexiblen Polymermaterial, einem Metalldraht, der in Röhrenmaterial eingebunden sein kann, sowie der eingeschlossenen und zu verdampfenden Substanz umfaßt. Das Material der Röhre kann aus unterschiedlichen Materialien bestehen, insbesondere Polyethylen.

Die GB 2 194 440 offenbart eine Sack oder Beutel enthaltend ein Luftreinigungsmittel. Der Beutel wird durch verschweißen oder verkleben zweier Kunststoffolien gebildet. Das Beutelmaterial besteht aus einem geschäumten Kunststoff, vorzugsweise Polyurethanschaum und weist Poren zum Auslaß des Mittels auf.

Die EP 0 835 666 offenbart eine Vorrichtung zur Freigabe von Duftstoffen. Die Vorrichtung besteht aus einer Tasche oder Behälter, dessen eine Seite wenigstens einen wasserdurchlässigen Abschnitt ab und welcher selbst nicht wasserlöslich ist. Der Wirkstoff ist ein trockenes Mittel, daß durch die Aufnahme von Wasser in eine Gelform überführt wird.

Die US-A-5,170,886 betrifft eine Verpackung bestehend aus vollständig versiegelten Plastikschalen zur Aufnahme eines abzugebenen Mittels, wie einem Deodorant. Zur Abgabe des Deodorants weisen die Schalen eine diffusionsoffene Wandung auf.

Gemäß einem ersten Aspekt der Erfindung (Anspruch 1) ist eine Anordnung zur Luftverbesserung vorgesehen, umfassend ein flüchtiges Luftverbesserungsmittel, das in einer permanent verschlossenen, jedoch zur betriebsmäßigen Wirkstoffabgabe diffusionsoffenen Umhüllung eingeschlossen ist, wobei die Umhüllung als beidseitig verschlossener Schlauchabschnitt ausgebildet ist, wobei das Material ein Silicon kautschuk oder eingetrochnetes Siliconacetat mit einer Gaspermeabilität von wenigstens 5x10⁻¹⁶m²s⁻¹Pa⁻¹ ist.

Der Einschluß des Luftverbesserungsmittels in einer permanent verschlossenen Umhüllung verhindert hierbei ein unerwünschtes Austreten des Mittels, welches daher problemlos auch in wachsartiger, zähflüssiger oder flüssiger Form vorliegen kann. Eine Einlagerung des Mittels in eine Matrix (imprägniertes Trägermaterial) ist hierbei entbehrlich. Für das eingesetzte Mittel ist die Umhüllung jedoch diffusionsoffen, d.h. das Mittel oder Wirkstoffe desselben werden betriebsmäßig nach Diffusion durch die Umhüllung hindurch zur Luftverbesserung abgegeben. Es erfolgt eine kontrollierte und anhaltende Freisetzung von Wirkstoff. Daher sind keine besonderen Dosiereinrichtungen notwendig, wie sie z.B. bei Duftsprays vorhanden sind. Die Anordnung kann wirkungsvoll eingesetzt werden, um z.B. Öl-, Farben-, Feuchtigkeits-, Schimmel- oder Nikotingeruch zu beseitigen. Die Ausbildung der Umhüllung aus einem Schlauchabschnitt gewährleistet eine einfache Herstellung, insbesondere kann die Umhüllung damit im wesentlichen einstückig hergestellt werden, bevorzugt ausgehend von einem Endlosschlauch. Der Herstellungsaufwand ist auch insofern relativ gering, weil es für den Einschluß des Mittels genügt, lediglich die beiden Enden des Schlauchabschnitts zu verschließen. Die Verwendung eines Schlauchabschnitts gewährleistet ein ansprechendes Erscheinungsbild und ermöglicht die Nutzung eines relativ großen Anteils des Umhüllungsmaterials als Diffusionspfad. Für den Hausgebrauch bevorzugt ist eine Duftkissenanordnung mit Abmessungen von einigen cm, beispielsweise mit einem Verhältnis der Länge des Schlauchabschnitts zum Durchmesser desselben im Bereich von etwa 0,5 bis 3.

Der Schlauchabschnitt ist aus einem Kunststoffmaterial gebildet, welches eine ausreichende Permeabilität für das Luftverbesserungsmittel bzw. Wirkstoffe desselben aufweist, nämlich Silicon-kautschuk oder eingetrochnetes Siliconacetat mit einer Gaspermeabilität von wenigstens 5x10⁻¹⁶ m² s⁻¹ Pa⁻¹. Bei Verwendung eines derartigen Materials kann die Umhüllung ohne Beeinträchtigung der Gebrauchseigenschaften problemlos mit einer Dicke von 0,3 mm oder mehr hergestellt werden, was die Umhüllung in mechanischer Hinsicht sehr widerstandsfähig macht. Auch ergibt sich mit den gewählten Materialien eine außerordentlich gute chemische Beständigkeit, was vor allem bei Anwendung im industriellen Bereich unter Umständen wesentlich ist. Schließlich weisen die gewählten Materialien eine sehr gute Wärmebeständigkeit auf, wodurch ein Luftverbesserungsmittel gegebenenfalls mit hoher Temperatur in den Schlauchabschnitt injiziert werden kann, etwa wenn der Schmelzpunkt für eine Injektion bei Raumtemperatur zu hoch ist (z.B. über 60°C).

Zum Verschließen des Schlauchabschnitts können beliebige für das gewählte Schlauchmaterial geeignete Techniken verwendet werden. Vorzugsweise ist der Schlauchabschnitt durch eine Verklebung und/oder eine Verschweißung und/oder eine Vulkanisierung des Schlauchmaterials verschlossen, wobei für die beiden Enden des Schlauchabschnitts auch unterschiedliche Verbindungstechniken eingesetzt werden können.

Das Mittel kann in den Schlauchabschnitt eingebracht werden, von dem noch kein Ende oder bereits ein Ende verschlossen wurde. Nachfolgend können dann die beiden Enden bzw. das andere Ende verschlossen werden. Herstellungstechnisch besonders günstig ist es jedoch, wenn das Mittel erst eingebracht wird, nachdem der Schlauchabschnitt beidseitig verschlossen wurde. Dies läßt sich durch eine Injektion des Mittels durch das Schlauchmaterial hindurch realisieren, wobei diese Injektion vollkommen unabhängig von der Herstellung des Schlauchabschnitts erfolgen kann, etwa als nachträglicher Bearbeitungsschritt an dem als Zwischenprodukt zuvor produzierten, bereits verschlossenen Schlauchabschnitt.

Bevorzugt enthält das Luftverbesserungsmittel einen Wirkstoff zur Bindung von Geruchsmolekülen. Derartige Wirkstoffe verbinden sich mit vorhandenen Geruchsmolekülen und neutralisieren diese. Schlechter Geruch kann dadurch vernichtet werden. Derartige Wirkstoffe können daher als "Geruchsvernichter" bezeichnet werden. Alternativ oder zusätzlich kann das Mittel auch einen Duftwirkstoff enthalten.

Vorteilhaft ist es, wenn das Luftverbesserungsmittel Natriumstearat und/oder Glyzerin und/oder Alkohol enthält. Damit lassen sich in einfacher Weise Geruchsvernichtungsmittel herstellen. Abhängig von der gewünschten Luftverbesserungswirkung und der damit verbundenen Wahl des Mittels ergibt sich in vielen Fällen bei Anwendungstemperatur (z.B. Raumtemperatur) eine zähflüssige bis flüssige Konsistenz des Mittels. Ferner kann ein gewähltes Mittel einen Schmelzpunkt aufweisen, der so niedrig ist, daß in der Praxis die Gefahr besteht, daß das Mittel sich verflüssigt. Bei der erfindungsgemäßen Anordnung ist dies aufgrund des permanenten Einschlusses des Mittels jedoch kein Problem, so daß jede gewünschte Zusammensetzung und Konsistenz des Luftverbesserungsmittels vorgesehen werden kann. Oftmals ist sogar die Verwendung eines Mittels mit niedrigem Schmelzpunkt im Hinblick auf die Wirksamkeit zu bevorzugen.

Im folgenden werden zwei Verfahren zur Herstellung einer Luftverbesserungsanordnung erläutert, von denen sich das erste besonders gut zur Herstellung einer Luftverbesserungsanordnung der oben beschriebenen Art eignet.

Gemäß einem weiteren Aspekt der Efindung ist ein Verfahren zur Herstellung einer Luftverbesserungsanordnung vorgesehen, umfassend die Schritte: Bereitstellen von Kunststoffschlauchmaterial, und Einschließen eines flüchtigen Luftverbesserungsmittels in beidseitig verschlossene, zur Wirkstoffabgabe diffusionsoffene Abschnitte des Schlauchmaterials.

Eine derart hergestellte Luftverbesserungsanordnung bietet die oben bereits erläuterten Vorteile.

Gemäß einem weiteren Aspekt ist ein Verfahren zur Herstellung einer Luftverbesserungsanordnung vorgesehen, umfassend die Schritte: Bereitstellen eines flüchtigen Luftverbesserungsmittels in zähflüssiger bis fester Phase, Beschichten des Luftverbesserungsmittels mit einem flüssigen oder zähflüssigen Kunststoffmaterial, und Verfestigung des Kunststoffmaterials zur Bildung einer zur Wirkstoffabgabe diffusionsoffenen Umhüllung.

Zum Bereitstellen des Luftverbesserungsmittels in der gewünschten Konsistenz kann eine zeitweilige Änderung der Konsistenz gegenüber der Konsistenz während des Gebrauchs vorgesehen sein, beispielsweise durch Zugabe von entsprechenden Additiven oder durch eine Temperaturbehandlung, insbesondere Abkühlung des Mittels. Entsprechendes gilt für eine gegebenenfalls notwendige Einstellung der Konsistenz des für die Beschichtung verwendeten Kunststoffmaterials. Die Verfestigung des Kunststoffmaterials, welches das Luftverbesserungsmittel umhüllt, kann bei Verwendung eines thermoplastischen Kunststoffs durch Abkühlung erfolgen. Auch ist eine Temperaturbehandlung zur Aushärtung eines Kunststoffs denkbar. Die Beschichtung des Luftverbesserungsmittels kann z B. durch kurzzeitiges Eintauchen in das Kunststoffmaterial oder durch Aufspritzen des Kunststoffmaterials erfolgen.

Die Erfindung wird nachfolgend anhand einiger Ausführungsbeispiele mit Bezug auf die beigefügten Zeichnungen detaillierter beschrieben. Dabei stellen dar:
- Fig. 1 und 2: eine Geruchsvernichterdose nach dem Stand der Technik,
- Fig. 3: eine Draufsicht auf eine erfindungsgemäße Luftverbesserungsanordnung in Form eines Duftkissens,
- Fig. 4: eine Seitenansicht des Duftkissens von Fig. 3 in Richtung des Pfeils IV in Fig. 3 betrachtet,
- Fig. 5: eine Seitenansicht des Duftkissens von Fig. 3 in Richtung des Pfeils V in Fig 3 betrachtet,
- Fig. 6: eine Schnittansicht des Duftkissens von Fig. 3 längs der Linie VI-VI in Fig. 3,
- Fig .7: eine Ausführungsform einer erfindungsgemäß hergestellten Luftverbesserungsanordnung in Form einer Duftkugel,
- Fig. 8: eine Zentralschnittansicht der Duftkugel von Fig. 7, und
- Fig. 9 bis 11: schematische Darstellungen zur Veranschaulichung der erfindungsgemäßen Herstellung des Duftkissens nach Fig. 3.

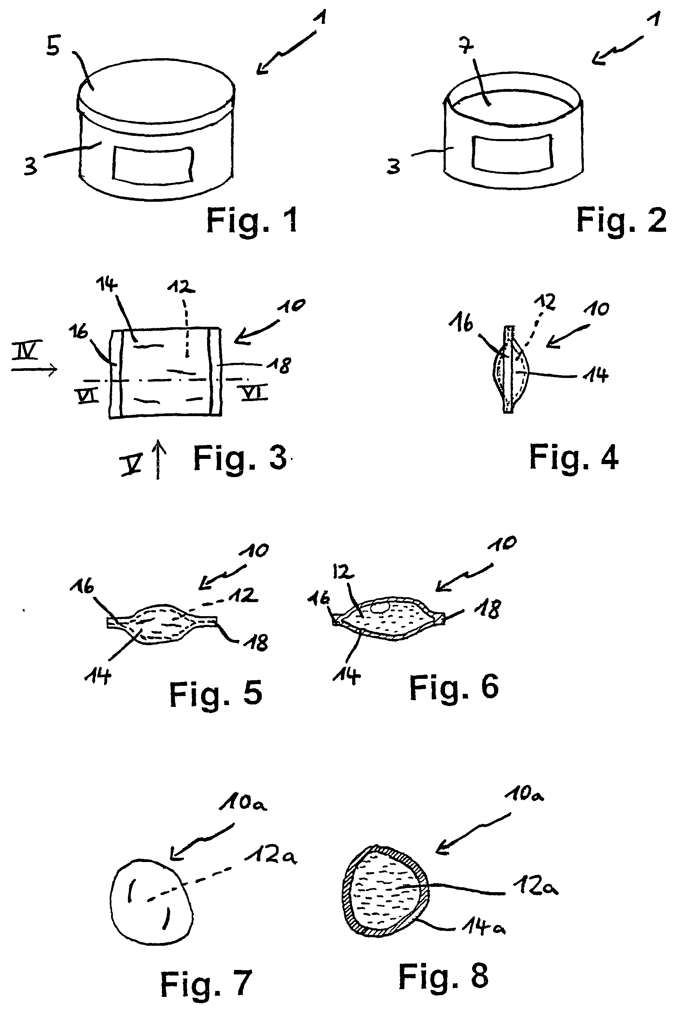

Fig. 1 zeigt einen herkömmlichen Geruchsvernichter 1 mit einer Dose 3 und einem aufgesetzten Deckel 5. Die Dose 3 ist mit einem wachsartigen Luftverbesserungsmittel 7 gefüllt, welches einen Wirkstoff zur Bindung von Geruchusmolekülen enthält. In der in Fig. 1 dargestellten Situation ist das Luftverbesserungsmittel 7 durch die Dose 3 und den Deckel 5 hermetisch eingeschlossen. Dose 3 und Deckel 5 sind aus Polypropylen hergestellt, so daß kein Wirkstoff entweicht.

Der Geruchsvermchter 1 arbeitet nach dem Verdunstungsprinzip. Nach Öffnung des Deckels 5 binden die Wirkstoffe des Mittels 7 die in der umgebenden Luft schwebenden Geruchsmoleküle und neutralisieren sie somit. Insbesondere bei Anwendung in mobiler Umgebung oder Umgebung mit erhöhter Umgebungstemperatur (z.B Auto, Boot etc.) besteht die Gefahr, daß das Mittel 7 ausläuft.

Die Fig. 3 bis 6 stellen ein erstes Ausführungsbeispiel einer erfindungsgemäßen Luftverbesserungsanordnung 10 dar, bei der ein Luftverbesserungsmittel 12 in einer Umhüllung in Form eines Siliconschlauchabschnitts 14 angeordnet ist, der an seinen beiden Enden 16, 18 jeweils mittels einer später beschriebenen Verschweißung/Vulkanisierung verschlossen ist. Für das somit permanent eingeschlossene Luftverbesserungsmittel 12 bildet der Siliconschlauchabschnitt 14 eine diffusionsoffene Umhüllung zur Abgabe von flüchtigen Luftverbesserungswirkstoffen.

Das Luftverbesserungsmittel 12 besitzt eine chemische Zusammensetzung (INCI) wie folgt: Aqua, Alcohol denat., Sodium Stearate, Glycerin, Sodium Cocoate, Parfum. Mit dieser Zusammensetzung wird eine besonders effiziente Luftverbesserungswirkung erreicht. Jedoch können auch andere geeignete Zusammensetzungen vorgesehen werden, insbesondere mit Wirkstoffen, die zur Produktion von Parfumen und Seifen üblich sind.

Das Material für den Siliconschlauchabschnitt 14 ist ein Silicon mit Methyl- und Vinylgruppen an der Polymerkette, etwa ein Siliconkautschuk der Klasse VMQ (Nomenklatur nach ASTM D1418). Das letztgenannte Material besitzt, gemessen bei 60 °C, eine Gaspermeabilität von etwa 3,3 × 10⁻¹⁶ m² s⁻¹ Pa⁻¹ (3,3x10⁻⁷ cm² s⁻¹ bar⁻¹) für Luft, etwa 2,8 × 10⁻¹⁶ m² s⁻¹ Pa⁻¹ (2,8 x10⁻⁷ cm² s⁻¹ bar⁻¹) für Stickstoff bzw. etwa 9,5 × 10⁻¹⁶ m² s⁻¹ Pa⁻¹ (9,5x10⁻⁷ cm² s⁻¹ bar⁻¹) für Kohlendioxid. Wenngleich in der Praxis die Permeabilität für die Wirkstoffe des Mittels 12 maßgeblich ist, so bilden diese angegebenen Werte, insbesondere bei Verwendung eines Siliconkautschuks, durchaus Materialparameter, die im Hinblick auf die Diffusion von Wirkstoffen der hier interessierenden Art von Bedeutung sind und damit bevorzugte Materialparameter darstellen.

Je nach Größe des zu verbessernden Luftvolumens und der vorhandenen Geruchsbelastung lassen sich die Füllmenge sowie die absolute Diffusionsrate geeignet wählen. Zweckmäßig ist in vielen Fällen eine Füllmenge von etwa 40 bis 200 g und eine Diffusionsrate unter Normalbedingungen von etwa 1 bis 10 g pro Tag. Die Diffusionsrate läßt sich durch entsprechende Wahl des Mittels 12, des Umhüllungsmaterials sowie der Dicke der Umhüllung einstellen. Die Wandungsdicke des Schlauchabschnitts 14 beträgt hier etwa 1 mm, so daß die davon gebildete Umhüllung z.B. durch scharfkantige oder spitze Gegenstande nicht leicht beschädigt werden kann. Aufgrund der besonderen Materialauswahl lassen sich sogar noch größere Wandungsdicken vorsehen und dabei Diffusionsraten in dem oben erwähnten, bevorzugten Bereich erzielen. Bei dem dargestellten Duftkissen 10 diffundieren etwa 2 g des Mittels 12 pro Tag durch die Umhüllung 14.

Die Fig. 7 und 8 stellen ein Ausführungsbeispiel einer erfindungsgemäß hergestellten Anordnung in Form einer Duftkugel 10a dar. Zur Herstellung dieser Duftkugel 10a wurde ein Luftverbesserungsmittel 12a unmittelbar nach dessen Herstellung in eine Form (hier: Kugelform) gegossen und abgekühlt. Nach Entnahme aus der Form wurde das Mittel kurzzeitig in flüssiges Siliconacetat eingetaucht und dadurch mit diesem Kunststoff beschichtet (Das Siliconmaterial läßt sich hierbei durch eine entsprechend eingestellte Formulierung flüssig vorsehen oder durch Zugabe eines Lösungsmittels verflüssigen). Schließlich wurde die damit gebildete Siliconumhüllung durch eine Trocknung verfestigt.

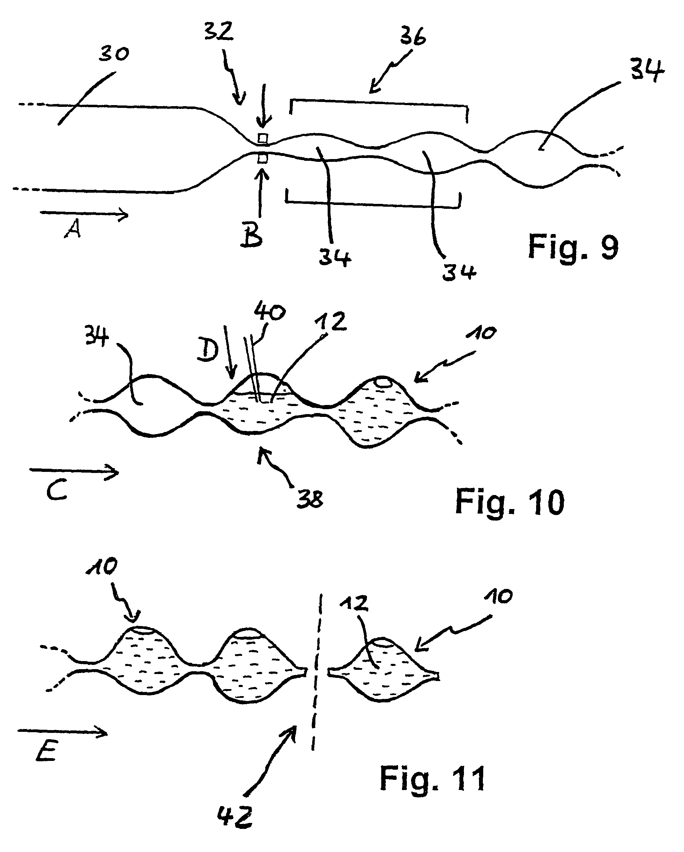

Die Fig. 9 bis 11 veranschaulichen die Herstellung des mit Bezug auf die Fig. 3 bis 6 beschriebenen Duftkissens 10.

In Fig. 9 erkennt man links ein extrudiertes Schlauchmaterial 30, welches sich in Richtung des Pfeils A bewegt und zunächst durch eine Schweißstation 32 läuft, an der das Schlauchmaterial an in Längsrichtung äquidistant befindlichen Stellen durch Druckbalken (Doppelpfeil B) zur Bildung jeweils beidseitig verschlossener Schlauchabschnitte 34 verschweißt wird.

Unmittelbar im Anschluß an das Verschweißen erfolgt ein Vulkanisieren des Siliconmatierials, indem das Material durch einen Ofen 36 läuft. Die Vulkanisierung dient im Falle von polymeren Kunststoffen dazu, den Vernetzungsgrad zu erhöhen und die aufeinanderfolgenden Schlauchabschnitte 34 sicher zu verschließen. Bei dem hier verwendeten Siliconmaterial tritt hierbei eine gewisse Aushärtung des Materials auf. Bei Verwendung eines anderen Materials, etwa eines thermoplastischen Kunststoffs, könnte eine derartige Aushärtung in entsprechend anderer Weise ablaufen.

Die Erwärmung des Schlauchmaterials und der eingeschlossenen Luft beim Durchlaufen des Ofens 36 bewirkt ein Aufblähen der einzelnen Schlauchabschnitte 34, so daß am Ende dieses Herstellungsschritts als Zwischenprodukt ein Strang miteinander verbundener, relativ großvolumiger Schlauchabschnitte 34 gebildet wird. Gewünschtenfalls lassen sich die einzelnen Schlauchabschnitte 34 in diesem Stadium vereinzeln. Bevorzugt ist jedoch, das Zwischenprodukt zu konfektionieren und gewünschtenfalls aufzuwickeln, um es später weiter zu bearbeiten, wie im folgenden beschrieben.

Fig. 10 veranschaulicht die Weiterbearbeitung des Strangs aus Schlauchabschnitten 34. Der Strang läuft in Richtung des Pfeils C durch eine Befüllstation 38, an der jeweils ein Schlauchabschnitt 34 durch eine Injektionsnadel 40 in Richtung des Pfeils D durchstochen wird und das über seinen Schmelzpunkt erwärmte Luftverbesserungsmittel 12 injiziert wird. Am Ende dieses Herstellungsschritts entsteht wieder ein Zwischenprodukt in Form eines Strangs miteinander verbundener, bereits mit dem Mittel 12 gefüllter Duftkissen 10.

Fig. 11 veranschaulicht die Vereinzelung der Duftkissen 10, die in Richtung des Pfeils E einer Schneidstation 42 zugeführt werden, welche den Duftkissenstrang im Bereich zwischen den einzelnen Duftkissen 10 durchtrennt. Alternativ könnte in diesem Bereich zwischen den einzelnen Duftkissen 10 auch eine Abreißperforation vorgesehen werden.

## Patentansprüche

1. Anordnung zur Luftverbesserung, umfassend ein flüchtiges Luftverbesserungsmittel, daß in einer permanent verschlossenen, jedoch zur betriebsmäßigen Wirkstoffabgabe diffusionsoffenen Umhüllung eingeschlossen ist, wobei das Material der Umhüllung eine Gaspermeabilität, gemessen für Luft bei 60 °C von wenigstens 5x10⁻¹⁶ m² s⁻¹ Pa⁻¹ (5x10⁻⁷ cm² s⁻¹bar⁻¹) bevorzugt wenigstens 2x10⁻¹⁵ m² s⁻¹ Pa⁻¹ (2x10⁻⁶ cm² s⁻¹ bar¹), aufweist und wobei weiterhin das Material ein Siliconkautschuk oder ein getrocknetes Siliconacetat ist.

2. Anordnung nach Anspruch 1, wobei das Umhüllungsmaterial als Schlauchabschnitt ausgebildet und ein Silikonkautschuk ist.

3. Anordnung nach Anspruch 2, wobei der Schlauchabschnitt (14) durch eine Verklebung des Schlauchmaterials verschlossen ist.

4. Anordnung nach einem der Ansprüche 2 oder 3, wobei der Schlauchabschnitt (14) durch eine Verschweißung des Schlauchmaterials verschlossen ist.

5. Anordnung nach einem der Ansprüche 2 bis 4, wobei der Schlauchabschnitt (14) durch eine Vulkanisierung des Schlauchmaterials verschlossen ist.

6. Anordnung nach einem der Ansprüche 2 bis 5, wobei das Luftverbesserungsmittel (12) nach beidseitigem Verschluß des Schlauchabschnitts (14) in den Schlauchabschnitt injiziert ist.

7. Anordnung nach einem der Ansprüche 2 bis 6, wobei die Umhüllung eine Wandungsdicke von etwa 0,3 bis 5 mm, bevorzugt etwa 0,5 bis 2 mm, aufweist.

8. Anordnung nach einem der Ansprüche 2 bis 7, wobei das Luftverbesserungsmittel (12) einen Wirkstoff zur Bindung von Geruchsmolekülen enthält.

9. Anordnung nach einem der Ansprüche 2 bis 8, wobei das Luftverbesserungsmittel (12) einen Duftwirkstoff enthält.

10. Anordnung nach einem der Ansprüche 2 bis 9, wobei das Luftverbesserungsmittel (12) Natriumstearat und/oder Glyzerin und/oder Alkohol enthält.

11. Anordnung nach einem der Ansprüche 2 bis 10, wobei das Luftverbesserungsmittel (12) einen Schmelzpunkt von weniger als 60°C, insbesondere etwa 30°C bis 50°C, besitzt.

12. Verfahren zur Herstellung einer Luftverbesserungsanordnung, nach einem der Ansprüche 1 bis 11, **gekennzeichnet durch** die Schritte:
- Bereitstellen von Kunststoffschlauchmaterial (30), und
- Einschließen eines flüchtigen Luftverbesserungsmittels (12) in beidseitig verschlossene, zur Wirkstoffabgabe diffusionsoffene Abschnitte (34) des Schlauchmaterials (30).

13. Verfahren nach Anspruch 12, umfassend die Schritte:
- Extrudieren eines Kunststoffschlauchs (30),
- Verschließen des Schlauchmaterials (30) in regelmäßigen Abständen längs des Schlauchmaterials zur Bildung jeweils beidseitig verschlossener Schlauchabschnitte (34),
- Injektion (38) des Luftverbesserungsmittels in die beidseitig verschlossenen Schlauchabschnitte (34) und Gewünschtenfalls Vereinzeln (42) der Schlauchabschnitte (34).

14. Verfahren nach Anspruch 13, wobei die Schlauchabschnitte (34) erst vereinzelt werden, nachdem das Luftverbesserungsmittel (12) injiziert wurde.

15. Verfahren nach Anspruch 12, 13 oder 14, wobei das Verschließen (32) der Schlauchabschnitte durch Verkleben und/oder Verschweißen und/oder Vulkanisieren des Schlauchmaterials (30) erfolgt.

16. Verfahren nach Anspruch 13, 14 oder 15, wobei das Verschließen (32) der Schlauchabschnitte durch Verschweißen und/oder Vulkanisieren unmittelbar im Anschluß an das Extrudieren des Schlauchmaterials (30) erfolgt.

17. Verfahren zur Herstellung einer Luftverbesserungsanordnung, nach Anspruch 1, **gekennzeichnet durch** die Schritte:
- Bereitstellen eines flüchtigen Luftverbesserungsmittels (12a) in zähflüssiger bis fester Phase,
- Beschichten des Luftverbesserungsmittels (12a) mit einem flüssigen oder zähflüssigen, und Silikonkautschuk oder Silikonacetat.
- Verfestigung des Silikonkautschuk oder Silikonacetat zur Bildung einer zur Wirkstoffabgabe diffusionsoffenen und permanent verschlossenen Umhüllung (14a).

18. Verfahren nach Anspruch 17, wobei das Beschichten des Luftverbesserungsmittels (12a) durch Eintauchen in den Silikonkautschuk oder Silikonacetat erfolgt.

## Claims

1. An arrangement for deodorising air, encompassing a volatile air reclaimer which is enclosed in a permanently sealed covering , however open for the diffusion of the operating active substance release, and where the covering material has a gas permeability measured for air at 60°C, of at least 5x10⁻¹⁶ m² s⁻¹Pa⁻¹(5x10⁻⁷ cm² s⁻¹bar⁻¹) preferably at least 2x10⁻¹⁵ m²s⁻¹ Pa⁻¹ 2x10⁻⁶ cm² s⁻¹bar¹) and further where the material is a silicone rubber or a dried silicone acetate.

2. An arrangement according to claim 1, where the covering material is constructed as a tube section and is a silicone rubber.

3. An arrangement according to claim 2, where tube section (14) is sealed by a gluing together of the tube material.

4. An arrangement according to any of claims 2 or 3, where tube section (14) is sealed by a bonding of the tube material.

5. An arrangement according to any of claims 2-4, where tube section (14) is sealed by a vulcanisation of the tube material.

6. An arrangement according to any of claims 2-5, where air reclaimer (12) is injected into the tube section after sealing both sides of the tube section (14).

7. An arrangement according to any of claims 2-6, where the covering has a wall thickness of about 0,3 to 5mm, preferably about 0,5 to 2mm.

8. An arrangement according to any of claims 2-7, where the air reclaimer (12) contains an active substance for bonding odour molecules.

9. An arrangement according to any of claims 2-8, where the air reclaimer (12) contains a fragrance active substance.

10. An arrangement according to any of claims 2-9, where the air reclaimer (12) contains sodium stearate and/or glycerine and/or alcohol.

11. An arrangement according to any of claims 2-10, where the air reclaimer (12) has a melting point of less than 60°C, in particular about 30°C to 50°C.

12. A method for producing an air deodorizing arrangement according to any of claims 1-11, **characterised by** the following steps:
- preparation of synthetic tube material (30) and
- enclosing a volatile air reclaimer (12) in sections (34) of the tube material (30), sealed on both sides and open for the diffusion of the active substance release.

13. A method according to claim 12, encompassing the steps:
- extruding a synthetic material tube (30),
- sealing the tube material (30) at regular intervals along the tube material for respectively forming tube sections (34) sealed on both sides,
- injecting (38) the air reclaimer into the section of tube (34) sealed on both sides and if desired, the singling out (42) of tube sections (34).

14. A method according to claim 13, where tube section (34) is singled out after the air reclaimer (12) has been injected.

15. A method according to claims 12,13 or 14, where the tube section sealing (32) results through gluing and/or bonding and/or vulcanisation of the tube material (30).

16. A method according to claims 13,14 or 15, where the tube section sealing (32) results through bonding and/or vulcanisation directly in connection with the extrusion of the tube material.

17. A method for producing an air deodorising arrangement, according to claim 1, **characterised by** the following steps:
- preparation of a volatile air reclaimer (12a) in a viscous to solid phase,
- coating the air reclaimer (12a) with a fluid or viscous, silicone rubber or silicone acetate,
- solidification of the silicone rubber or silicone acetate to form a permanently sealed coating (14a) and open for diffusion of the active substance release.

18. A method according to claim 17, where coating the air reclaimer (12a) results through immersion in the silicone rubber or silicone acetate.

## Revendications

1. Système pour désodoriser l'air, comprenant un désodorisant volatile enfermé dans une enveloppe fermée en permanence, mais uniquement ouverte pour diffuser un agent actif pendant son utilisation, le matériau de l'enveloppe présentant une perméabilité au gaz, mesurée pour l'air à 60°C, d'au moins 5x10⁻¹⁶ m² s⁻¹Pa⁻¹ (5x10⁻⁷ cm² s⁻¹bar⁻¹), de préférence d'au moins 2x10⁻¹⁵ m²s⁻¹Pa⁻¹ (2x10⁻⁶ cm²s⁻¹bar⁻¹), et le matériau étant en outre un caoutchouc siliconé ou un acétate de silicone séché.

2. Système selon la revendication 1, le matériau d'enveloppe présentant la forme d'un tronçon de flexible et étant un caoutchouc siliconé.

3. Système selon la revendication 2, le tronçon de flexible (14) étant fermé par collage du matériau de flexible.

4. Système selon l'une quelconque des revendications 2 ou 3, le tronçon de flexible (14) étant fermé par soudage du matériau de flexible.

5. Système selon l'une quelconque des revendications 2 à 4, le tronçon de flexible (14) étant fermé par vulcanisation du matériau de flexible.

6. Système selon l'une quelconque des revendications 2 à 5, le désodorisant (12) étant injecté dans le tronçon de flexible après fermeture du tronçon de flexible (14) aux deux extrémités.

7. Système selon l'une quelconque des revendications 2 à 6, l'enveloppe présentant une épaisseur de paroi de 0,3 à 5 mm environ, de préférence de 0,5 à 2 mm environ.

8. Système selon l'une quelconque des revendications 2 à 7, le désodorisant (12) contenant un agent actif pour lier des molécules odorantes.

9. Système selon l'une quelconque des revendications 2 à 8, le désodorisant (12) contenant un agent actif odorant.

10. Système selon l'une quelconque des revendications 2 à 9, le désodorisant (12) contenant du stéarate de sodium et/ou de la glycérine et/ou de l'alcool.

11. Système selon l'une quelconque des revendications 2 à 10, le désodorisant (12) présentant un point de fusion inférieur à 60°C, notamment de 30°C à 50°C.

12. Procédé de fabrication d'un système pour désodoriser l'air selon l'une quelconque des revendications 1 à 11, **caractérisé par** les étapes :
- mettre à disposition le matériau de flexible en matière plastique (30), et
- enfermer un désodorisant (12) volatile dans des tronçons (34) du matériau de flexible (30) fermés aux deux extrémités, ouverts pour diffuser un agent actif pendant l'utilisation.

13. Procédé selon la revendication 12, comprenant les étapes :
- extruder un flexible en matière plastique (30),
- fermer le matériau de flexible (30) à des distances régulières le long du matériau de flexible pour former des tronçons de flexible (34) respectivement fermés aux deux extrémités,
- injecter (38) le désodorisant dans les tronçons de flexible (34) fermés aux deux extrémités, et le cas échéant séparer (42) les tronçons de flexible (34).

14. Procédé selon la revendication 13, les tronçons de flexible (34) n'étant séparés qu'après l'injection du désodorisant (12).

15. Procédé selon la revendication 12, 13 ou 14, la fermeture (32) des tronçons de flexible étant réalisée par collage et/ou soudage et/ou vulcanisation du matériau de flexible (30).

16. Procédé selon la revendication 13, 14 ou 15, la fermeture (32) des tronçons de flexible étant réalisée par soudage et/ou vulcanisation directement après l'extrusion du matériau de flexible (30).

17. Procédé de fabrication d'un système pour désodoriser l'air, selon la revendication 1, **caractérisé par** les étapes :
- mettre à disposition un désodorisant (12a) volatile en phase visqueuse à l'état solide,
- enrober le désodorisant (12a) par un liquide ou par un caoutchouc siliconé visqueux, ou par un acétate de silicone,
- solidifier le caoutchouc siliconé ou l'acétate de silicone pour former une enveloppe (14a) ouverte pour diffuser un agent actif et fermée en permanence.

18. Procédé selon la revendication 17, l'enrobage du désodorisant (12a) étant réalisé par trempage dans le caoutchouc de silicone ou l'acétate de silicone.
